(19) Europäisches Patentamt
European Patent Office
Office européen des brevets

(11) Publication number: **0 243 849 A2**

(12) **EUROPEAN PATENT APPLICATION**

(21) Application number: **87105834.3**

(22) Date of filing: **21.04.87**

(51) Int. Cl.³: **C 07 C 50/06**
**C 07 C 50/14**

(30) Priority: **23.04.86 IL 78611**

(43) Date of publication of application:
**04.11.87 Bulletin 87/45**

(84) Designated Contracting States:
**AT BE CH DE ES FR GB GR IT LI LU NL SE**

(71) Applicant: **YEDA RESEARCH AND DEVELOPMENT COMPANY, LIMITED**
**P.O. Box 95**
**Rehovot 76100(IL)**

(72) Inventor: **Keinan, Ehud**
**Efal Street, 30**
**Holon(IL)**

(72) Inventor: **Eren, Doron**
**Taran Street, 19**
**Rehovot(IL)**

(74) Representative: **Vossius & Partner**
**Siebertstrasse 4 P.O. Box 86 07 67**
**D-8000 München 86(DE)**

(54) **Process for the production of polyprenoid type compounds.**

(57) There is provided a simple and economic process for the production of polyprene type compounds such as ubiquinones, vitamin K1, vitamin K2, plastoquinones and solanesol type compounds. The process comprises gradually building up stepwise a polyprenoid moiety while same is attached to a terminal group, or building up such polyprenoid moiety and coupling it to a desired terminal group.

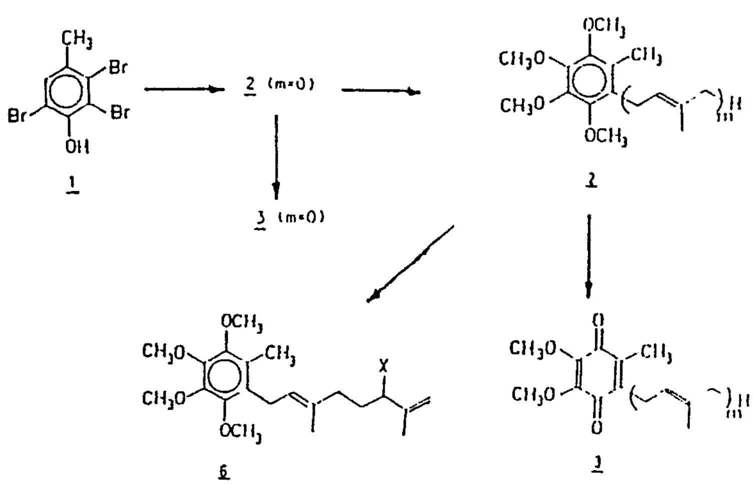
1
2 (m=0)
3 (m=0)
2
6
3

Figure 1

EP 0 243 849 A2

0243849
21. April 1987

Our Ref.: W 445EP

Case: T/571

YEDA RESEARCH AND DEVELOPMENT CO., LTD.

PROCESS FOR THE PRODUCTION OF POLYPRENOID TYPE COMPOUNDS

The invention relates to the production of polyprenoid type compounds , such as various ubiquinones, vitamin K1 type compounds, vitamins K2, plastoquinones and solanesol type compounds, hereinafter also named as polyprene compounds.

There is provided a novel process for the production of the polyprene compounds which are part of the desired compounds. There is further provided a novel simple process for the production of the ubiquinone moiety, 2,3-dimethoxy-5-methyl-6-substituted-1,4- benzoquinone, where the 6-substituent is generally a polyprenyl substituent, and in the case of Ubiquinone (0) the 6 position is occupied by a hydrogen atom.

Ubiquinones are a family of 2,3-dimethoxy-5-methyl-6-polyprenyl-1,4 -benzoquinones, which are widely distributed in nature, of the general formula

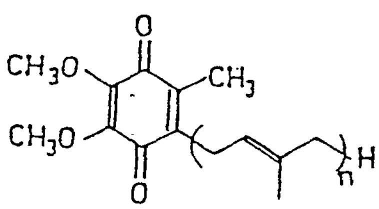

wherein n is zero, of 1 to 12.

Ubiquinones and other quinones with polyprenyl side chains are found in high concentrations in mitochondria, microsomes, chloroplasts and chromatophores. In the membranes of these organelles they play very important roles - promoting electron transfer in photosynthesis and respiratory chains, acting as antioxidants and lipid-soluble vitamins etc. Reports on activity of ubiquinones and related compounds as fertility factors, in inhibition of human platelet aggregation, in stabilization of biological membranes, in enhancement of immune response to antigenic stimulation.

in resistance to bacterial infections an in inhibition of the generation of leucotrienes in sensitized lung tissue, have been published. Q10 is used in Japan as a drug believed to improve cardio-vascular function and suppress some cardiotoxic side effects of cancer chemotherapy. Pharmacological actions at the molecular level are still unclear.

Various rather complicated routes of synthesis are known for the production of polyprene compounds. The known routes of synthesis of ubiquinones are cumbersome multi-step processes, which result in expensive products. The present invention provides a simple controlled stepwise oligomerization process for preparing a polyprene compound of any desired chain length.

All synthetic approaches, since the first synthesis by Folkers in 1958, are based on a separate synthesis of the aromatic nucleus and a derivative of the side chain and their subsequent coupling. Attempts to couple a polyprenyl alcohol to the protected hydroquinone have resulted in low yields owing to the instability of the allylic alcohol in acid: the best yield reported for such a Friedel-Crafts approach using a polymeric acid as the condensing catalyst and decaprenol as the alcohol is 50%. unfortunately, decaprenol is too expensive to be used with such low yields.

Another approach was based on nickel chemistry - conversion of the prenyl bromide to a nucleophilic $\pi$-allyl nickel complex which was cross coupled to a bromo-quinone.

A third and better approach was based on the (relatively) readily available nonaprenyl alcohol - solanesol. The quinone was first coupled to isoprene epoxide. the allylic alcohol thus formed was converted to a bromide and then reacted with the anion of solanesyl-p.tolyl sulfone.

A semisynthetic approach was based on modification of ubiquinone 7 which was extracted form yeast. The protected quinone was manipulated to give th

terminal chloride. The anion of farnesyl-p.tolylsulfone was coupled to that and sulfone and protecting groups removed.

In most of those routes the quinone part (ubiquinone-O) was synthesized from vanilline in several steps. Other approaches, based on gallic Acid or trihydroxybenzene are also known.

All those routes are long and complicated and have low overall yields. They also depend on relatively expensive starting materials.

There is provided a process for he production of a variety of polyprene compounds. The process is applicable for the production of a number of vitamin K compounds, of plastoquinones, ubiquinones and of other polyprene compounds.

The process of the invention involves the preparation of polyprene compounds of desired chain length, a process for the coupling of the thus produced compounds with a "terminator" which is a compound of the benzoquinone type or other, according to the desired final product, and a process for the production of said "terminator".

The polyprene compounds are produced by a transition metal controlled oligomerization, and it is possible to obtain any desired chain length. i.e. any required number of isoprene groups in the chain.

The polyprene compounds are prepared by the activation of an initiator by means of a suitable activator, such as a base,*) by the activation of a monomer by a suitable activator, such as Pd(O) catalyst,*) the interaction of the activated compounds with each other resulting in an oligomer, and repeating such reaction of activated oligomer with further monomer until the desired chain length is obtained.

Fig 1 is a reaction scheme setting out the production of the key intermediate 2 from compound 1, together with the production of compound 6.

Fig. 2 is a reaction scheme setting out the production of key intermediates 4 and 5. and their subsequent conversion to the corresponding ubiquinone 3.

*) see page 12a

This reaction proceeds via intermediates 6 and 7.

Fig. 3 sets out the total synthesis of coenzyme Q10.

Fig. 4 illustrates some representative examples of the type of compounds which can be prepared according to the present invention.

Fig. 5 is a schematic illustration of two alternative stepwise oligomerization procedures for the production of polyprenoid type compounds to the general formula I. S in this figure means a p-tolyl sulfone moiety and E a methyl ester moiety.

Fig. 6 is another reaction scheme of stepwise oligomerization.

According to a preferred embodiment there is provided a A process for the production of polyprenoid type compounds of the general formula I:

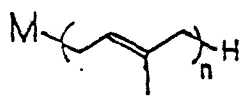

I

wherein M is selected from:

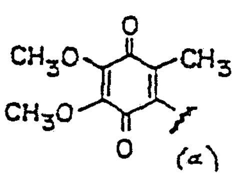
(a)

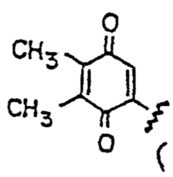
(b)

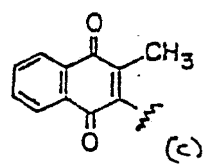
(c)

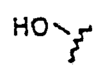
(d)

and wherein n is zero or an integer of from 1 to 13, and when n=0,1,2 M is different from (d). This process comprises activating compound II comprising one or more isoprene units (n=0 or an integer) wherein M is as defined above and X is hydroxy, halogen, alkanoate, carbonate, tosylate, phosphate, or any other conventional leaving group. Activating compound III comprising one or more isoprene units (n=0 or an integer) substituted at the 1 position with electron withdrawing group or groups and where X is hydroxy, halogen, alkanoate, carbonate, tosylate, phosphate, or any other conventional leaving group. Reacting these activated compounds with each other, and if required repeating the reaction

with another type III compound until the required chain length is obtained, coupling the resulting compound with a terminator of the general formula IV wherein A and A' are as defined above

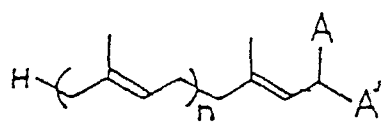

IV

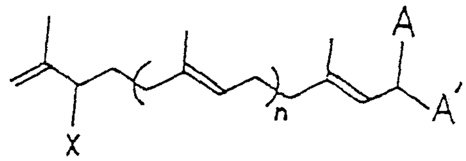

III

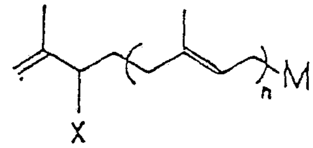

II

to yield an intermediate of the general formula V:

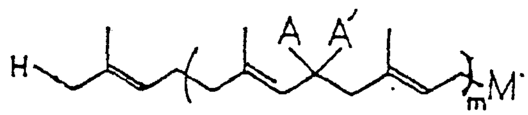

V

where m is an integer of from 1 to 7 and A and A' are electron-withdrawing-groups or A is an electron-withdrawing-group and A' is a hydrogen and M is as defined above; removing A and A' and deprotecting the M moiety to result in the desired product 1; or, alternatively, starting by coupling a compound IV to a compound III. repeating with more III, as required, and terminating in a similar manner by coupling to a II compound, removing A and A' and deprotecting the M moiety to result in 1.

In a preferred process according to the invention the type II compound is of the formula:

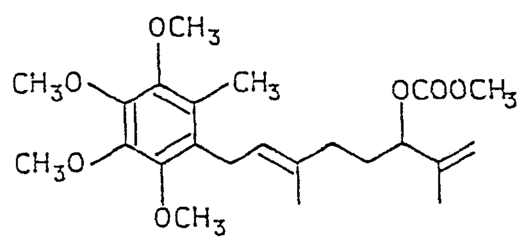

and a process according to the invention where the type III compound is of the formula:

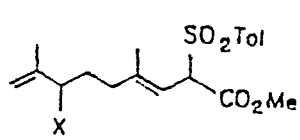

and a process according to the invention where the type IV compound is of the formula:

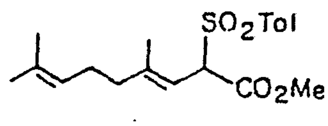

Tol = p-$CH_3$-$C_6H_5$

The invention is illustrated by way of example with reference to the preparation of vitamins Q0, Q2, Q4, Q6 and Q10.

**Example 1:**

**The synthesis of Ubiquinone 0**

The synthesis is according to the reaction scheme

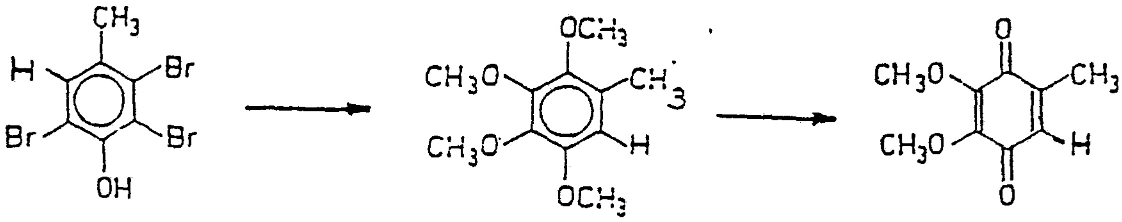

1 2 3

**Stage A**

**1,2,6-Tribromo-4-methylphenol, 1.** prepared according to Th. Zinke , Liebigs Annalen der Chemie **320** p. 205, 1902) p-Cresol (108 g, 1 mol) was dissolved in $CHCl_3$ (120 ml) containing iron powder (4 g, 0.071 mol). Bromine (500 g, 3.12 mol) was then added dropwise over 5 hrs at room temperature. The solution was stirred for additional 48 hrs and then filtered, washed with dilute aqueous $NaHSO_3$ and dried over $MgSO_4$. The solvent was removed under reduced pressure, and the residue was recrystallized from hexane to yield 1 (262 g, 7).

**Stage B**

**2,3,4,5-Tetramethoxy-5-methylbenzene, 2** (n=0). Sodium (2.3 g, 100 mmol)

was dissolved in methanol (40 ml) DME (30 ml) and dimethylcarbonate (5 ml) were added and most of the methanol (30 ml) was removed by distillation. CuCN (1.5 g, 16.7 mmol) was introduced into the mixture and a solution of 1 (3.44 g, 10.3 mmol) in 10 ml DME was added dropwise over 3 hrs while the temperature was kept at 80°C. The mixture was stirred for 5 more hrs at this temperature, followed by addition of water (80 ml), cooling to 50°C and dropwise introduction of dimethylsulfate (10 ml). The mixture was stirred at room temperature for an additional 2 hrs and conc aqueous $NH_4OH$ (25 ml) was added. The mixture was extracted with $CH_2Cl_2$, and the organic extract was washed with dilute aqueous HCl and water and dried over $MgSO_4$. The solvent was removed under reduced pressure, giving tetramethoxytoluene, **2** (1.98 g, 94%).

Stage C

Vitamin Q0, **3**. (Prepared according to J. Mlochowsky, Tetrahedron Letters 36, p. 123, 1979). Pyridine-2,6-dicarboxylic acid (3.34 g, Fluka) was added to a cold (0°C) solution of **2** (1.70 g, 8.0 mmol) in 40 ml of 7:3 acetonitrile:water. A cold (0°C) solution of ceric ammonium nitrate (CAN) (10.96 g, 20 mmol) in 40 ml of 1:1 acetonitrile:water was slowly added over 20 min, and the mixture was stirred for 20 min at 0°C and for 10 min at room temperature. The reaction mixture was poured into 40 ml of water and extracted with $CH_2Cl_2$. The organic solution was dried over $MgSO_4$, followed by solvent removal under reduced pressure and column chromatography (silica gel, hexane:ethyl acetate 20:1), giving **3** (1.22 gr, 84%) in the form of red crystals.

Example 2:

The Synthesis of Ubiquinone 2.

The synthesis is according to Figure 1.

Stage A

(2'E)-1-(3',7'-Dimethylocta-2',6'-dienyl)-6-methyl-2,3,4,5-tetra

methoxybenzene, 2(m=2).

*n*-Butyllithium (30 ml of 1.1 M solution in hexane) was added over 30 min under argon to a solution of 2(m=0) (4.9 g, 23.1 mmol) in 50 ml hexane containing TMEDA (5 g) at 0°C. The mixture was stirred for additional 30 min, followed by addition of THF (250 ml) and CuCN (3 g, 33 mmol). After stirring for 30 min, a solution of geranyl bromide (5 g, 23 mmol) in THF (50 ml) was added slowly over 60 min, and the mixture was stirred for additional 60 min. The reaction was then quenched with saturated aqueous $NH_4Cl$, ether (250 ml) was added, and the organic phase was separated and washed with aqueous $NH_4OH$, water and brine and dried over $MgSO_4$. The solvent was removed under reduced pressure and the residue chromatographed over silica gel (hexane:ethyl acetate 25:1) to give 2(m=2) as a colorless oil (5.3 g, 66 %).

Stage B

Vitamin Q2, 2(m=2)

Oxidation of 2(m=2) with CAN according to the procedure described above for preparation of ubiquinone-0. gave ubiquinone-2 3(m=2) in 87% yield (2.21 g). In a similar manner the coupling of other polyprenoid-type compounds can be carried out to obtain compounds wherein M has the significance as defined herein and in the claims.

Example 3:

The Synthesis of Ubiquinone 6.

The synthesis is according to Figures 1 and 2.

Stage A

Methyl 4,8 dimethyl-2-(p. tolyl-sulfonyl)-3,7-nonadienoate, 4. Geranyl-p. tolylsulfone (prepared according to S. Terao et. al. J.C.S Perkin Trans. I 1978 p.1101) (6.5 g ) was dissolved in 40 ml dry DMF to which 10 ml of dry dimethyl carbonate was added. The solution was cooled to -20° and potassium t–butoxide (5g)

was added. The mixture was stirred at -20 for 2 hrs and at room temperature for an additional hour. The reaction was quenched with sat. ammonium chloride solution. The mixture was poured into 250 ml water and extracted with ether (4x20 ml). the extracts were combined, washed with HCl 1 M, water and brine, dried over MgSO4 and the solvent was removed under reduced pressure. the residue was purified by chromatography (silica gel, 10% EtOAc in hexane to give 7.41 g of 4 as colorless oil (95% yield).

**Stage B**

**Methyl 7-Hydroxy-4,8 dimethyl-2-(p. tolyl-sulfonyl)-3,7-nonadienoate, 5.**
To a cold (0°) solution of geranyl p.-tolylsulfone (2.92 g ,10 mmol) in 50 ml $CH_2Cl_2$ was added dropwise over 30 minutes a solution of 2 gr. mCPBA in 50 ml $CH_2Cl_2$. The reaction mixture was stirred for additional 15 minutes and then transferred to a separation funnel, washed with sat. $NaHCO_3$ solution and water and dried over $MgSO_4$, The solvent was removed under reduced pressure and the residue was dissolved in 15 ml dry toluene and 20 ml 0.5M aluminum triisopropylate in isopropanol. The solution was refluxed for 20 hours, cooled, washed with 20 ml HCl 2M and water, dried over $MgSO_4$ the solvent was removed under reduced pressure, and the residue dissolved in DMF (50 ml) and dimethyl carbonate (20 ml). The solution was cooled to 0°C and potassium t-butoxide (4 g) was added slowly. After stirring for 1 hour the solution was allowed to warm to room temperature. After additional stirring for 1 hour the reaction mixture was poured into saturated $NH_4Cl$ solution. extracted with ether, washed with dil. HCl. water and brine and dried over $MgSO_4$. The solvent was removed under pressure and the residue dissolved in 0.5 M sodium methylate in methanol solution. After stirring at room temperature for 4 hours the solution was neutralized with HCl, the methanol was removed under reduced pressure, the residue partitioned between ether and water. The ether phase was separated, dried over $MgSO_4$. The solvent was removed under reduced pressure and the residue chromatographed (silica gel.

35% EtOAc in hexane to give 5(X=OH) 2.01 g, 55%).

Stage C

**6-(6-Methylcarbonate-3,7-dimethylocata-2,7-dienyl)-1-methyl-2,3,4,5-tetramethoxybenzene, 6.** Compounds 6 was prepared from compound 3 (n=2) the same way compound 5 was prepared from geranyl sulfone except for the last methanolysis step which was omitted.

Stage D

**6-(9,17-dicarbomethoxy-9,17-di(p.tolylsulfonyl)-3,7,11,15,19,23-hexamethyltetracosa-2,6,10,14,18,22-hexaenyl)-1,2,3,4-tetramethoxy-5-methylbenzene, 7(n=2).** Compound 6 (420 mg) and compound 5 (x=OH, 440 mg) were dissolved in THF (5 ml), $Pd(P\phi_3)_4$ (58 mg) was added and the solution stirred for 2 hours at room temperature. KCN (20 mg) and water (1 ml) were added and the mixture stirred for an additional 15 minutes. The solution was then added to ether (30 ml), washed with water and brine, dried over $MgSO_4$ and the solvent was removed under reduced pressure. The residue was chromatographed (silica gel , 35% EtOAc in hexane). The product was dissolved in benzene ( 5 ml) and diethylanilline (0.5 ml) pyridine (2 drops) and methylchloroformate (0.5 ml) were added in this order. After stirring for 3 hours the solution was washed (in a separating funnel) with 1 M HCl, water, brine, dried over MgSO4 and the solvent was removed under reduced pressure. The residue was dissolved in THF (5 ml) and reacted with compound 4 (420 mg) in the presence of $Pd(P\phi_3)_4$ (58 mg). The reaction mixture was worked up as described and chromatographed (silica gel 20% EtOAc in hexane gave compound 7 (825 mg, 79%). (Note: Ø = phenyl)

Stage E

**(2'E,6'E,10'E,14'E,18'E)-1-(3',7',11',15',19',23'-hexamethyl-9',17'-di(p.tolylsulfonyl tetracosa-2',6',10',14',18',22'-hexaenyl)-2,3,4,5-tetramethoxy-6-methylbenzene 7(n=2.** A solution of 7(n=2) (347 mg, 0.33 mmol), 4-aminothiophenol (64 mg) and cesium carbonate (35 mg) in 3 ml DMF was stirred at 85$^{o}$ for 1 hr. The

mixture was then poured into water (20 ml) and extracted with water (3X10 ml). The combined extracts were washed twice with 1M HCl (5 ml), water and brine and dried over $MgSO_4$ The solvent was removed under reduced pressure to yield 2 in the form of a nearly colorless oil which was found to be pure by TLC, NMR and elemental analysis (225 mg, 98%).

Stage F

**(2'E,6'E,10'E,14'E,18'E)-1-(3',7',11',15',19',23'-hexamethyl-tetracosa -2',6',10',14',18',22'-hexaenyl)-2,3,4,5-tetramethoxy-6-methylbenzene, 2(n=2. (Done according to Mohri et al, Chemistry Letters 1985 451.** Compound 7(n=2) (127 mg, 0.18 mmol) was dissolved in dry THF (2 ml) containing 5 mg $Pd(DPPP)Cl_2$. The solution was cooled to $0^o$ and a solution of triethylborohydride (0.4 ml, 1.0M, 0.4 mmol) was added dropwise over 2 hours. The mixture was stirred for additional 3 hours and then poured into diisopropyl ether (20 ml). The ether solution was washed with 1 *M* NaCN solution (5 ml), water, brine and dried over $MgSO_4$. The solvent was removed under reduced pressure and the product purified by TLC (20% EtOAc in hexane) to give 2(n=2) (64 mg, 0.16 mmol. 88%).

Stage G

Vitamin Q6, 3(n=2). Compound 2(n=2) was deprotected as described in Example 1, Stage C. The product was purified by chromatography (silica gel, 15% EtOAc in hexane) to yield vitamin Q6.

Example 4:

The Synthesis of Ubiquinone 4.

Stage A

(2'E,6'E,10'E)-1-(3',7',11',15'-tetramethyl-9'-(p.tolylsulfonyl) hexadeca-2',6',10',14'-tetraenyl)-2,3,4,5-tetramethoxy-6-methylbenzene 7(n=1, A'=H). $Pd(DPPE)_2$ (17 mg) and potassium t.-butoxide (60 mg) were

added to a solution of 6 (126 mg, 0.30 mmol) and geranyl p.tolyl sulfone (150 mg) in dry THF (5 ml). After being stirred at reflux for 4 hours the solution was cooled, the reaction was quenched with saturated aqueous $NH_4Cl$ and KCN, the solvent was decanted, the flask washed three times with $CH_2Cl_2$ and the washings combined with the THF solution. the combined solution was dried over $MgSO_4$, the solvents were removed under reduced pressure and the residue chromatographed (20% EtOAc in toluene) over silica gel to give compound 7(n=1, A'=H) (106 mg, 55%).

**Example 5:**

**The synthesis of Ubiquinone 10.**

The synthesis is similar to that described in Example 3 except that in stage D the coupling with compound 5 was repeated three times before coupling to compound 4.

In a manner similar to that described above in Examples 2 and 3, starting with compound

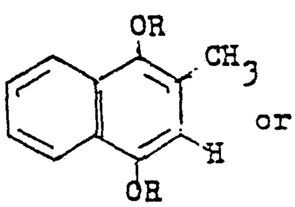
or
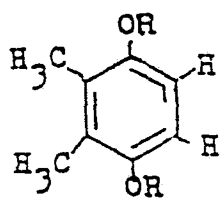

derived from menadione or from dimethylbenzoquinone, respectively, wherein R is lower alkyl, and coupling same with a suitable polyprene compound under the same reaction conditions, there are obtained compounds defined by general formula I on page 4, where M designates the groups 2 and 3, respectively defined at the top of page 5. The results were confirmed by elemental analysis and NMR spectroscopy.

Bases useful as activators are NaH (sodium hydride), KOtBu (potassium-tert.-butoxide), BSA (N,O-bis-(trimethylsilyl)-acetamide;
DBU 1,8-diazabicyclo/5,4,0/-undec-7-ene ;
DBN 1,5-diazabicyclo/4,3,0/-non5-ene (Trost , B.M., Acc. Chem. Res. 13, 1980, 385; Trost, B.M.; Verhoven,T.R., "Comprehensive Organometalli Chemistry", 8, 1982, 779).

Suitable Pd(o) catalysts are $Pd(PPh_3)_4$; $Pd(dppe)_2$; $Pd_2(dba)_3 + PR_3$; $Pd/\overline{p}(OR)_3/_4$; $PdCl_2$ + DIBAL-H + dppe, "Pd-Ⓟ" Ⓟ = polystyrene.
The above are mentioned in the following references:
Trost et al., J.Am.Chem.Soc. 102, 1980, 5979-5981; Trost, B.M. and Keinan, E J.Am.Chem.Soc. 100, 1978, 7779.

CLAIMS

1. A process for the production of polyprenoid type compounds of the general formula I:

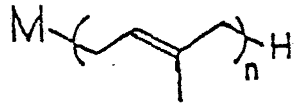

I

wherein M is selected from:

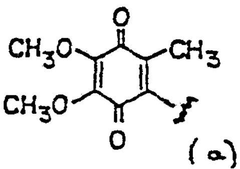 (a) 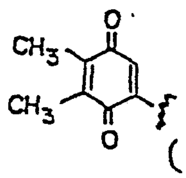 (b) 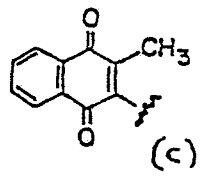 (c) 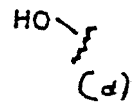 (d)

and wherein n is zero or an integer of from 1 to 13, and when n=zero M is different from (d); and compounds II, III, IV and V are as described on pages 4 and 5; which comprises activating a compound II. activating a compound III, reacting these two activated compounds with each other, and if required repeating the reaction with another III until the required chain length is obtained. and coupling the resulting adduct with a compound IV to yield an intermediate of the general formula :

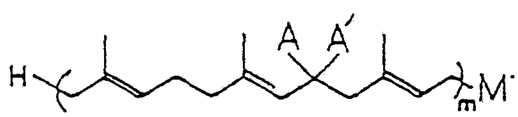

where m is an integer of from 1 to 7 and A and A' are electron-withdrawing-groups and M is as defined above; removing A and. A' and deprotecting the M moiety to result in the desired product; or, alternatively, starting by coupling a compound IV to a compound III. repeating with more III. as required, and terminating in a similar manner by coupling to a II compound.

2. A process according to claim 1 where compound II (being either IIA or IIB) is of the general formula:

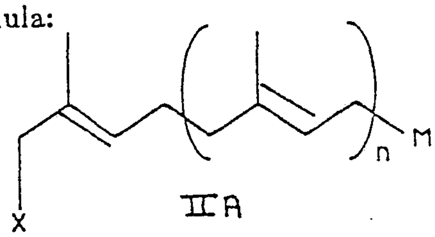

IIA

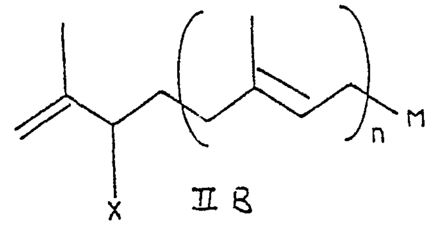

II B

comprises one or more isoprene units, one of which is substituted by an X, where X is halogen, hydroxyl, alkanoate, carbonate, tosylate, phosphate or any other conventional leaving group, n is zero or an integer and M is as defined in claim 1;

3. A process according to claim 1 where compound III of the general formula:

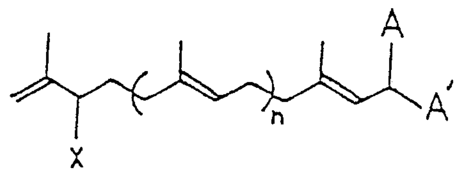

comprises one or more isoprene units substituted at the 1-position with tw electron-withdrawing-groups, A and A', n is zero or an integer and with an X defined in claim 2.

4. A process according to claim 1 where compound IV comprises one or mo isoprene units substituted at the 1-position with two electron-withdrawing-groups, and A', of the general formula:

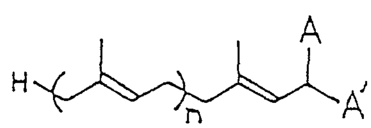

where n is zero or an integer.

5. A process according to any of claims 1 to 4, wherein the activation of the I and IV compounds is effected with base.

6. A process according to any of claims 1 to 5, wherein the activation is by means of a palladium(0) catalyst, results in an intermediate of the general formula:

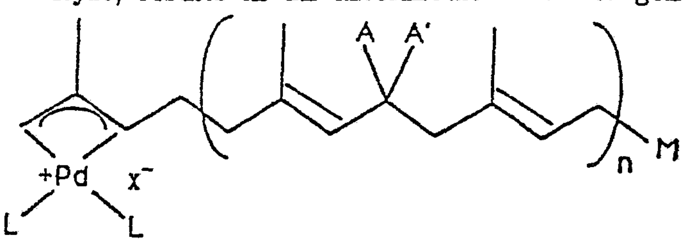

where A and A' are as defined in claim 1, and L is a neutral nitrogen- or phosphorous-containing ligand.

7. A process according to any of claims 1-6, wherein I(a), n=0, is prepared by dissolving sodium methoxide in DME/methanol, adding CuCN, adding 2,3,6-tribromo-4-methyl phenol in DME at elevated temperature, adding dimethyl sulfate and working up by conventional means, resulting in 2,3,4,5-tetramethoxytoluene, which is converted to the desired compound by oxidation with ceric ammonium nitrate (CAN).

8. A process according to any of claims 1 to 7, wherein M is:

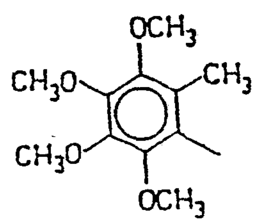

wherein some of the said II compounds are prepared by direct metalation of tetramethoxytoluene, followed by subsequent transition-metal-mediated coupling to the appropriate polyprenyl type compound of the general structure I, where M is a conventional leaving group.

9. A process according to any of claims 1 to 7, wherein A is a strong electron-withdrawing-group and A' is a hydrogen.

10. A process for the production of polyprenoid and ubiquinone-type compounds, substantially as herein before described, with reference to the examples and reaction schemes.

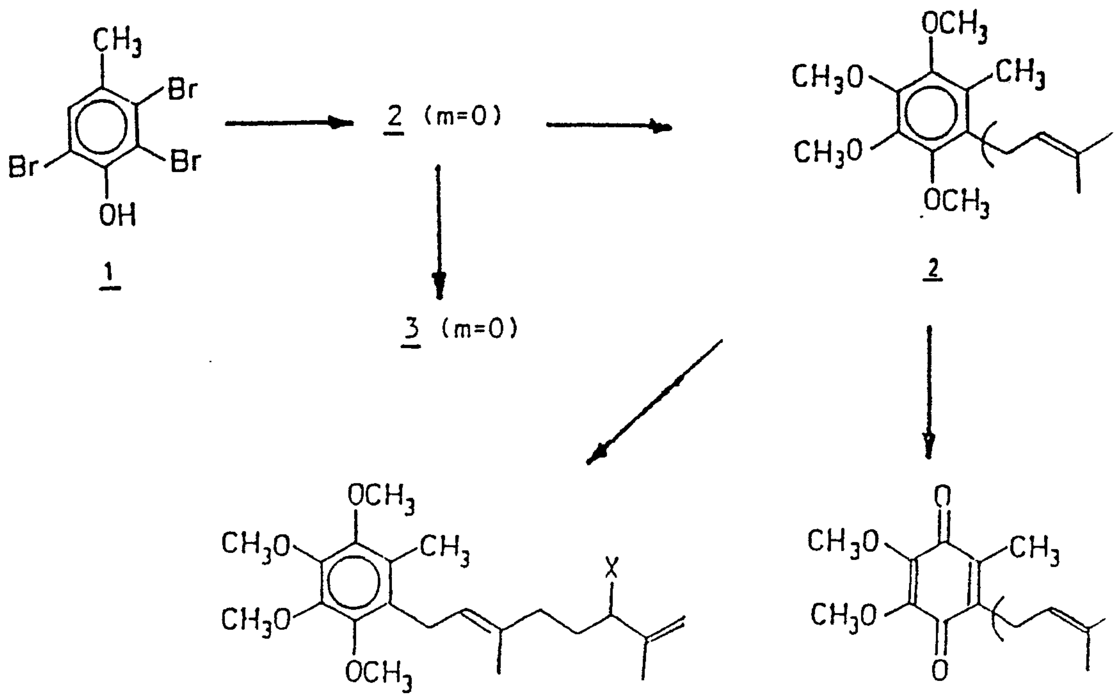

1

2 (m=0)

3 (m=0)

2

6

3

Figure 1

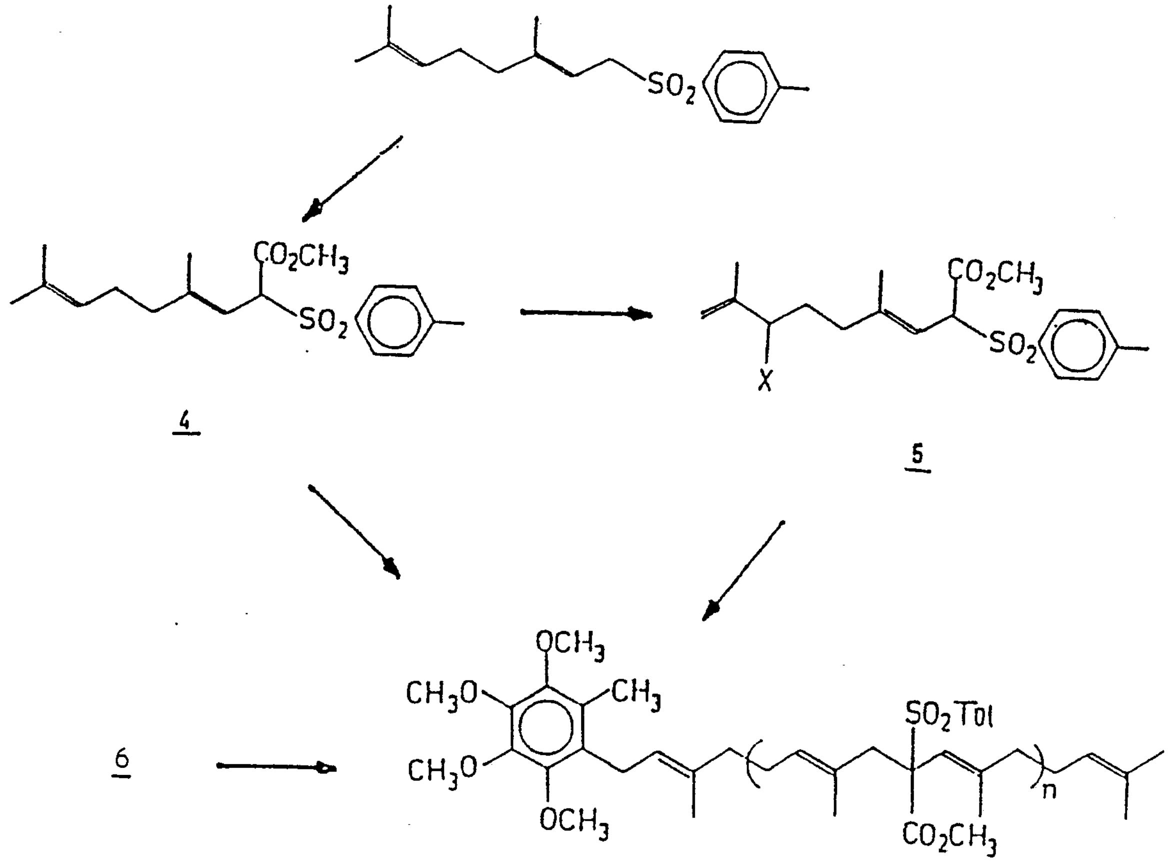

4

5

6

7

Figure 2

3

Total Synthesis of Coenzyme $Q_{10}$

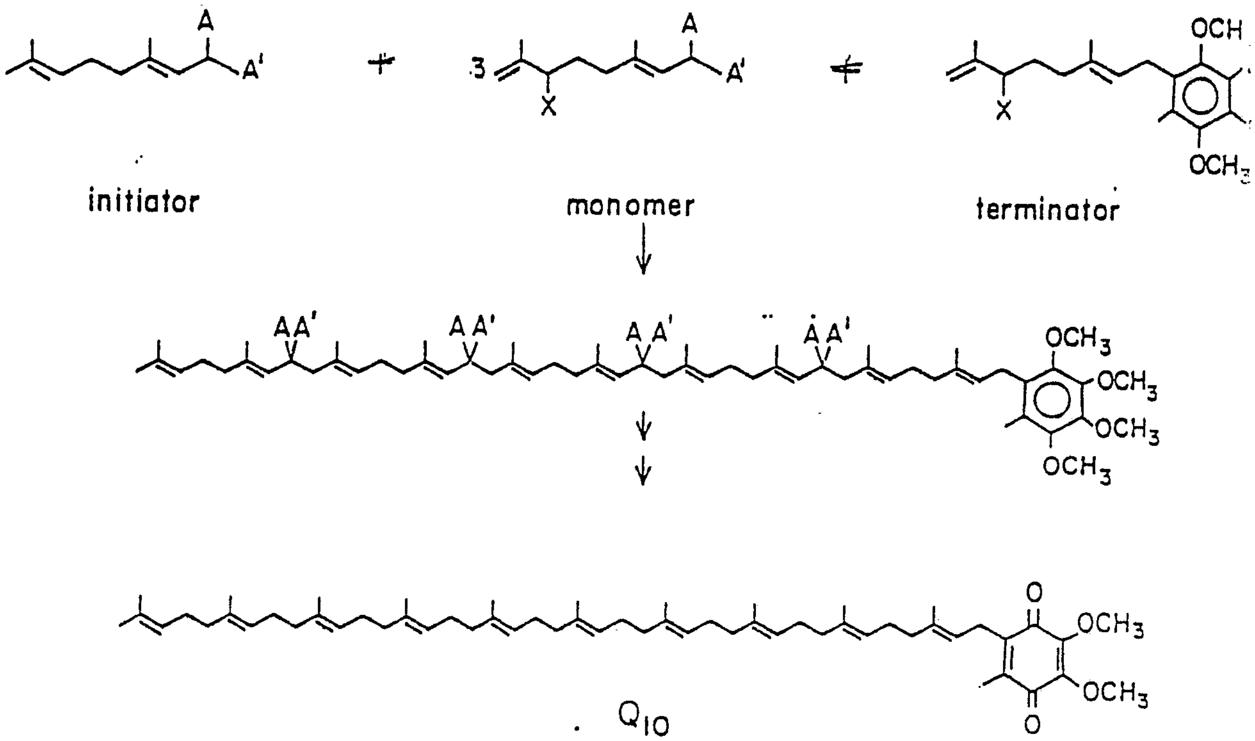


Figure 3

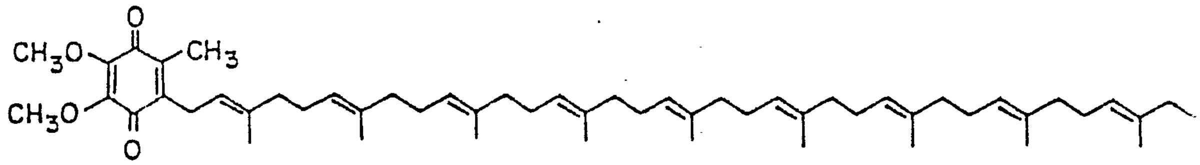

Coenzyme $Q_{10}$

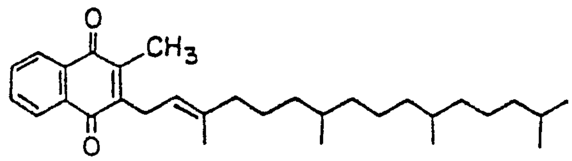

Vitamin $K_1$

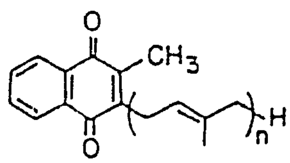

n=1-13

Vitamin $K_2$

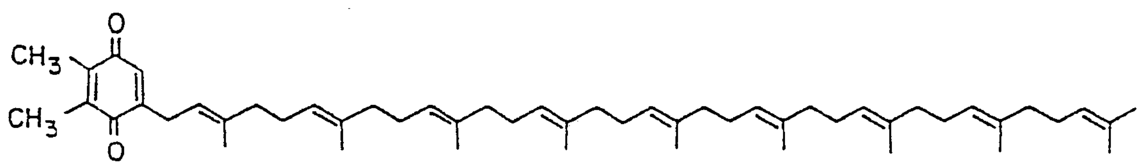

Plastoquinone-9

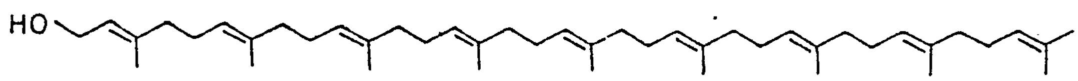

Solanesol

Figure 4

A

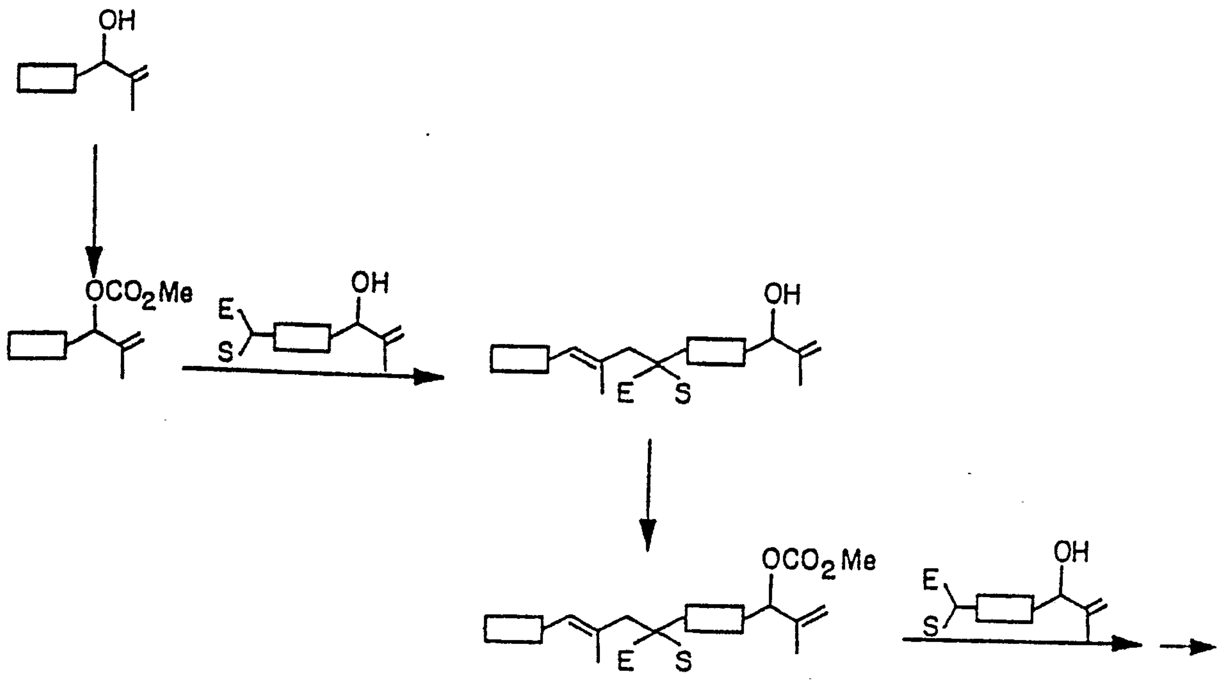

B

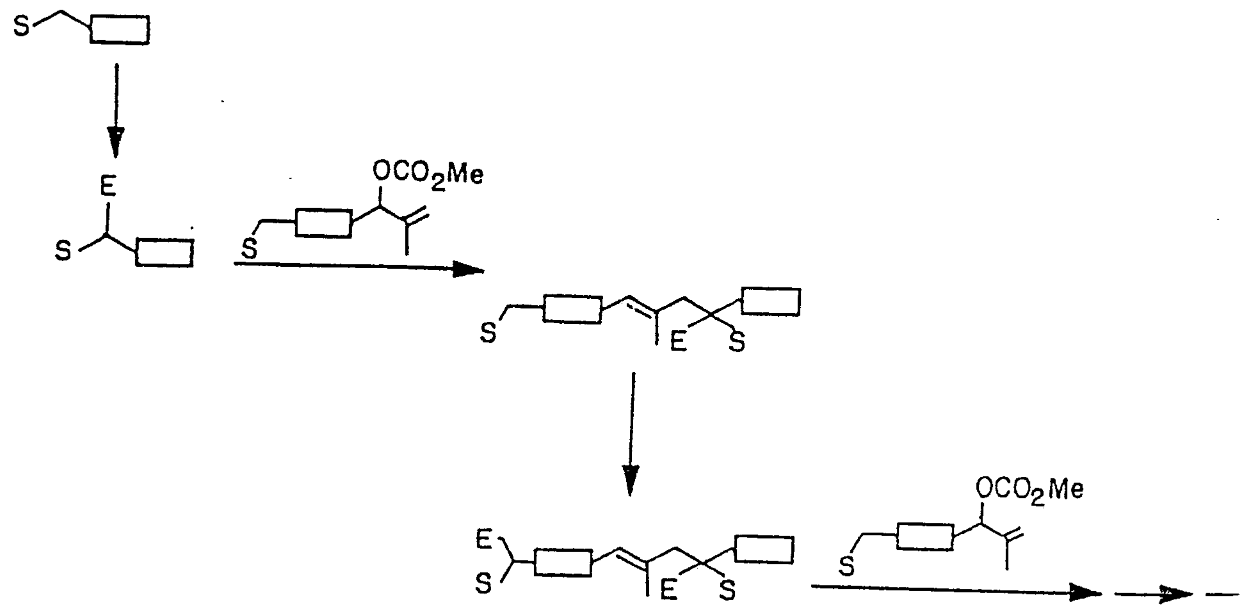

Figure 5

02438

6

Figure 6

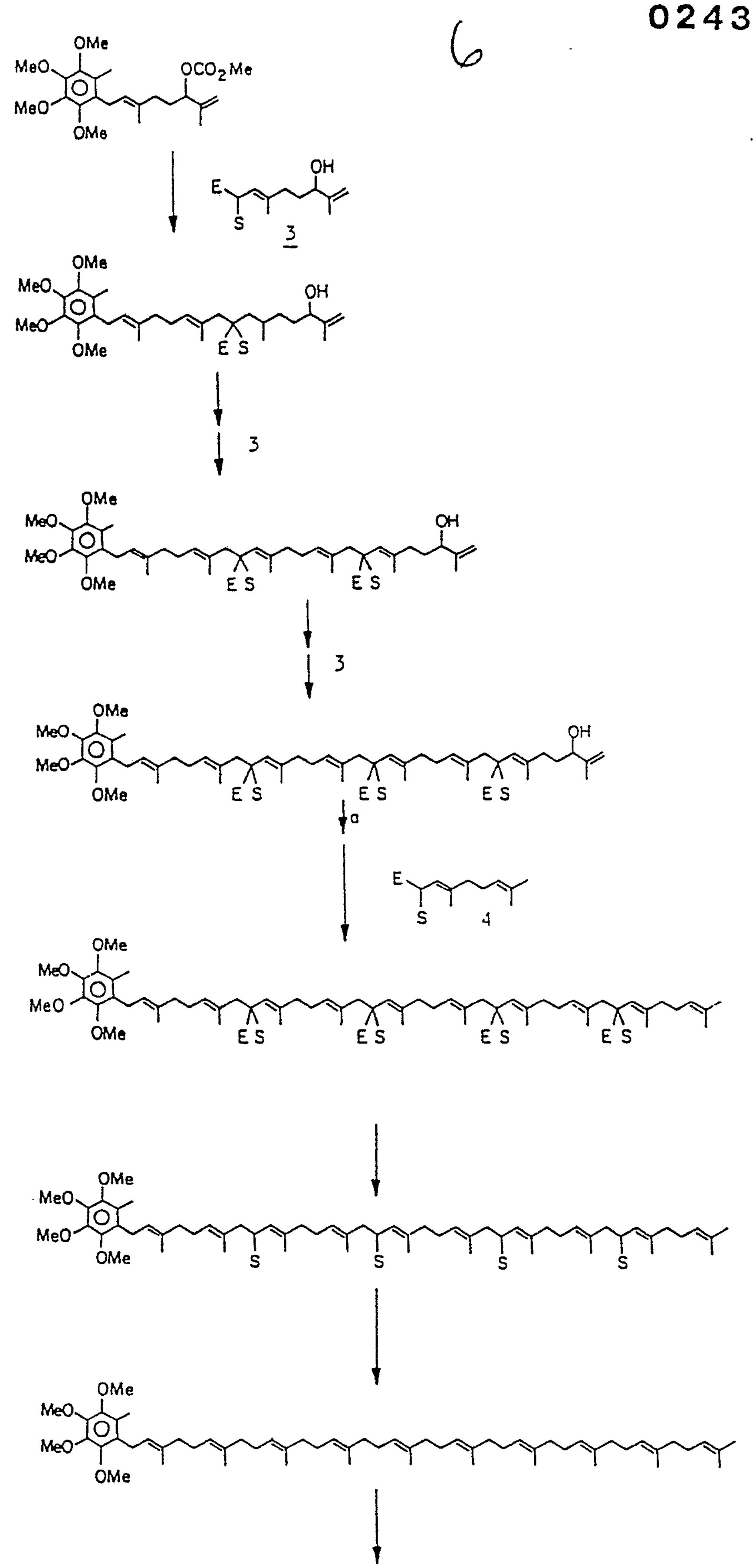


Coenzyme $Q_{10}$